(19) Europäisches Patentamt
European Patent Office
Office européen des brevets

(11) Numéro de publication: **0 472 670 B1**

# (12) FASCICULE DE BREVET EUROPEEN

(45) Date de publication de fascicule du brevet: **29.03.95**

(51) Int. Cl.[6]: **C07K 5/06**, C07K 5/08, C07K 5/10

(21) Numéro de dépôt: **91902104.8**

(22) Date de dépôt: **31.12.90**

(86) Numéro de dépôt internationale : **PCT/FR90/00975**

(87) Numéro de publication internationale : **WO 91/12263 (22.08.91 91/19)**

## (54) BIPEPTIDES SYMETRIQUES COMPORTANT UN RESTE POLYALKYLENEGLYCOL, PROCEDE DE PREPARATION ET UTILISATION DANS LE DOSAGE DES PROTEASES.

(30) Priorité: **19.02.90 FR 9001964**

(43) Date de publication de la demande: **04.03.92 Bulletin 92/10**

(45) Mention de la délivrance du brevet: **29.03.95 Bulletin 95/13**

(84) Etats contractants désignés: **DE**

(56) Documents cités:
**EP-A- 0 025 190**
**EP-A- 0 280 610**
**WO-A-87/00056**

(73) Titulaire: **SERBIO**
**Parc d'Activités Economiques,**
**Parispace 3,**
**125, avenue Louis Roche**
**F-92230 Gennevilliers (FR)**

(72) Inventeur: **QUENTIN, Gérard**
**145, rue des Renouillers**
**F-92700 Colombes (FR)**
Inventeur: **MARTINOLI, Jean-Luc**
**2, rue du Tremble**
**F-92390 Villeneuve-la-Garenne (FR)**

(74) Mandataire: **Clisci, Serge et al**
**S.A. FEDIT-LORIOT & AUTRES**
**38, avenue Hoche**
**F-75008 Paris (FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

## DOMAINE DE L'INVENTION

La présente invention concerne, en tant que produits industriels nouveaux, les composés bipeptidiques de formule I ci-après. Elle concerne également leur procédé de préparation et leur utilisation dans le domaine du dosage d'enzymes appartenant à l'ensemble des protéases et peptidases, en particulier en tant que substrats dans le domaine du dosage quantitatif de tels enzymes notamment les enzymes de la classe E.C. 3.4.4 (à présent nouvelle classe "E.C. 3.4.21", selon l'ouvrage "Enzyme Nomenclature", Elsevier Scientific Publishing Company, Amsterdam 1973, pages 238 et suivantes).

## ART ANTERIEUR

On sait que les enzymes de la classe sus-visée sont des substances qui scindent les liaisons amides du squelette protéique ou peptidique du côté du groupe carboxyle des restes Arg, Lys, Orn et His. Il s'agit là d'un mécanisme de clivage bien connu de l'homme du métier et qui est amplement exemplifié dans les documents antérieurs cités ci-après.

Les substrats d'enzymes de ladite classe actuellement utilisés sont des composés essentiellement tri- ou tétrapeptidiques dont l'extrémité N-terminale est généralement substituée par un groupe de blocage tel que benzoyle, benzyloxycarbonyle, t-butyloxycarbonyle, t-amyloxycarbonyle, tosyle, acétyle ou analogue, et dont l'extrémité C-terminale est amidifiée par un groupe aminé pouvant être un reste radioactif ou un reste capable de conférer, avant ou (mieux) après clivage, une coloration ou une fluorescente notamment un groupe p-nitroaniline. Voir à cet effet, les documents FR-A-2 546 163, FR-A-2 372 798, EP-A-0 004 256, US-A-4 508 644, US-A-4 448 715, FR-A-2 471 411, FR-A-2 317 280 et FR-A-2 459 226.

Il se trouve que les dérivés peptidiques antérieurement connus ont peu d'affinité pour l'eau. Ils sont peu solubles ou peu dispersables dans l'eau, par suite il est quelquefois nécessaire de leur adjoindre un solvant organique pour pouvoir les utiliser. Les systèmes comprenant des solvants mixtes ne sont guère compatibles, d'une manière générale, avec les milieux biologiques : ils entraînent soit une diminution de l'activité du substrat, soit encore, dans certains cas, une détérioration de l'enzyme que l'on veut doser. De plus, la faible affinité pour l'eau de ces substrats entraîne une diminution de la sensibilité des méthodes de dosage des enzymes.

On connaît de FR-A-2 546 163 précité une solution technique pour améliorer l'hydrosolubilité des substrats enzymatiques selon laquelle le peptide comporte sur l'extrémité N-terminale un groupe polyhydroxycarbonyle.

On connaît également de EP-A-0 025 190 un composé polypeptidique comprenant un reste de polyéthylèneglycol monoéthérifié par un groupe alkyle (cf. page 8, lignes 6-8), à savoir le produit de formule

$Me\text{-}PEG_{600}\text{-}CO\text{-}(CH_2)_2\text{-}CO\text{-}Val\text{-}Arg\text{-}Val\text{-}pNA$

(voir page 10, ligne 35), ou $PEG_{600}$ désigne le reste de polyéthylèneglycol ayant un poids moléculaire de l'ordre de 600 environ.

On connaît enfin de EP-A-0 280 160 des substrats dipeptidiques dont l'extrémité N-terminale comporte un reste alkyloxycarbonylalkylènecarbonyle tel que le reste méthyloxymalonyle.

## SOLUTION DE L'INVENTION

Selon l'invention, on préconise une nouvelle solution technique, qui est différente de celle proposée par FR-A-2 546 163, d'une part, et de celle envisagée par EP-A-0 025 190, d'autre part, en ce sens qu'elle fait appel à un compose comprenant deux chaînes monoaminoacides ou peptidiques qui sont chacune liées par leur extrémité N-terminale à un groupe polyalkylèneglycol divalent par l'intermédiaire d'un pont carbonyle CO.

Plus précisément, cette nouvelle solution technique repose sur le choix d'un groupe polyalkylèneglycol appartenant à l'ensemble des polyéthylèneglycols et polypropylèneglycols lié à deux chaînes de monoaminoacide ou deux chaînes peptidiques, chaque extrémité O-terminale du reste polyalkylèneglycol étant reliée par l'intermédiaire d'un groupe CO à l'extrémité N-terminale d'une chaîne monoacide ou peptidique pour conférer l'hydrosolubilité ou l'hydrodispersabilité nécessaire pour la sensibilité des dosages d'enzymes. Le fait que le reste polyalkylèneglycol est lié à deux chaînes augmente la sensibilité du dosage.

**OBJET DE L'INVENTION**

Suivant l'invention, on préconise de nouveaux composés appartenant à l'ensemble des aminoacides et des peptides contenant un reste d'aminoacide relié au reste d'un polyalkylèneglycol et ayant une grande affinité pour l'eau, lesdits composés, qui sont différents par leur structure des peptides antérieurement connus notamment en tant que substrats enzymatiques, étant caractérisés en ce qu'ils sont choisis parmi l'ensemble constitué par :

(i) les aminoacides et peptides symétriques de formule :

R-A-B-CO-Q-CO-B-A-R (I)

dans laquelle

Q est un reste de polyalkylèneglycol choisi parmi les restes de polyéthylèneglycol et polypropylèneglycol de formule :

$-O[CH(X)CH_2O]_n-$

où X est H ou Me, et n est un nombre entier supérieur ou égal à 1,

B représente une simple liaison, un reste de monoaminoacide ou une chaîne peptidique comprenant de 2 à 9 restes monoaminoacides,

A est un reste de monoaminoacide basique choisi parmi l'ensemble comprenant les restes d'α-aminoacides comportant un groupe basique sur une chaîne latérale,

R est un moyen de marquage permettant la révélation de la substance enzymatique à identifier ou doser; et

(ii) leurs sels d'addition.

**ABREVIATIONS**

Dans la présente description, on a utilisé les abréviations suivantes par commodité :

- <u>pour les restes d'aminoacide</u>

| | |
|---|---|
| ACC | = 1-amino-cyclohexane-1-carbonyle |
| AHX | = ε-aminohexanoyle |
| Aib | = 2-aminoisobutyryle (ou 2-méthylalanyle) |
| Ala | = α-alanyle |
| β-Ala | = β-alanyle |
| Arg | = arginyle |
| Asn | = asparaginyle |
| Asp | = α-aspartyle |
| β-Asp | = β-aspartyle |
| ATC | = thiazolidine-4-carbonyle (ou thioprolyle) |
| Aze | = azétidine-2-carbonyle |
| Abu | = 2-aminobutyryle |
| 4Abu | = 4-aminobutyryle |
| CHA | = 3-cyclohexylalanyle |
| CHG | = α-cyclohexylglycyle |
| CHT | = 3-(4-hydroxycyclohexyl)alanyle |
| Cle | = cycloleucyle (ou 1-amino-cyclopropane-1-carbonyle) |
| Cys | = cystéyle |
| Dbu | = 2,4-diaminobutyryle |
| Gln | = glutamyle |
| Glu | = glutaminyle |
| γ-Glu | = γ-glutaminyle |
| Gly | = glycyle |
| His | = histidyle |
| 3Hyp | = 3-hydroxyprolyle (ou 3-hydroxy-2-pyrrolidinecarbonyle) |
| 4Hyp | = 4-hydroxyprolyle (ou 4-hydroxy-2-pyrrolidinecarbonyle) |
| Ile | = isoleucyle |
| Leu | = leucyle |

Lys = lysyle
MeGly = N-méthylglycyle (ou sarcosyle)
Met = méthionyle
Nle = norleucyle
Nva = norvalyle
Orn = ornithinyle
Phe = phénylalanyle
Phg = phénylglycyle
Pip = pipécolinoyle
Pro = prolyle
Pyr = pyroglutaminyle (ou 2-pyrrolidone-5-carbonyle)
Ser = séryle
Thr = thréonyle
Tyr = tyrosyle
Val = valyle

- pour les autres abréviations :

Ac = acétyle
AcOH = acide acétique
Adoc = adamantyloxycarbonyle
Aoc = t-amyloxycarbonyle
Boc = t-butyloxycarbonyle
Bu = n-butyle
Bop = hexafluorophosphate de 1-(benzotriazolyl)oxy-tris(diméthylamino)phosphonium (autre nomenclature réactif de CASTRO) de formule

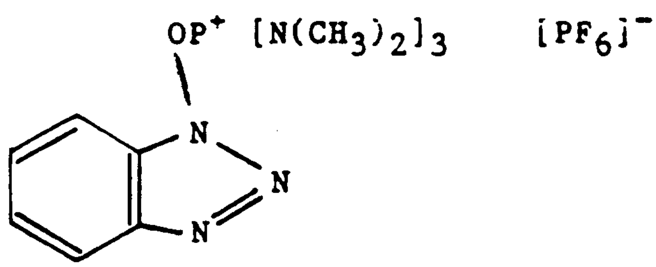

Bz = benzoyle
Bzl = benzyle
Cbo = carbobenzoxy
CCM = chromatographie sur couche mince
o-Cl-pNA = o-chloro-p-nitroanilino [ou (2-Cl)pNA]
DCCI = dicyclohexylcarbodiimide
DCHU = dicyclohexylurée
DIEA = diisopropyléthylamine
DMF = diméthylformamide
Et = éthyle
$Et_3N$ = triéthylamine
EtO = éthoxy
Fmoc = 9-fluorènylméthyloxycarbonyle
Foc = furfuryloxycarbonyle
HMPT = N,N,N',N',N",N"-hexaméthylphosphorotriamide
HOBT = 1-hydroxybenzotriazole
H-TFA = acide trifluoroacétique (ou encore HTFA)
Iboc = isobornyloxycarbonyle
iBu = isobutyle
iPr = isopropyle
Me = méthyle
MeO = méthoxy
Mor = 1-morpholinyle

| | | |
|---|---|---|
| OD | = | densité optique |
| OSu | = | N-oxysuccinimide ou (2,4-dioxopyrrolidin-1-yl)oxy |
| PAG | = | polyalkylèneglycol |
| PEG | = | polyéthylèneglycol |
| Ph | = | phényle |
| pH | = | cologarithme de la concentration en ions $H^+$ |
| Pi | = | 1-pipéridinyle |
| PM | = | poids moléculaire |
| pNA | = | p-nitroanilino ou $(4\text{-}NO_2)C_6H_4NH$ |
| PPG | = | polypropylèneglycol |
| Pr | = | n-propyle |
| Py | = | 1-pyrrolidinyle |
| RT | = | température ambiante (15-20°C) |
| tBu | = | t-butyle |
| TEA | = | triéthanolamine |
| THF | = | tétrahydrofuranne |
| Tos | = | p-toluènesulfonyle (ou tosyle) |
| Z | = | benzyloxycarbonyle |
| Z(p-Cl) | = | p-chlorobenzyloxycarbonyle |
| Z(p-OMe) | = | p-méthoxybenzyloxycarbonyle. |

## DESCRIPTION DETAILLEE DE L'INVENTION

Le groupe Q est un reste de polyalkylèneglycol (PAG). Il comprend les restes de polyéthylèneglycol (PEG) quand X = H et de polypropylèneglycol (PPG) quand X = Me. Dans le groupe Q chaque extrémité O-terminale est reliée à l'extrémité N-terminale de l'une des deux chaînes monoaminoacides ou peptidiques par un groupe carbonyle. De façon avantageuse, le reste PAG aura un poids moléculaire moyen compris entre environ 60 et environ 1000 et de préférence compris entre 200 et 600. De façon préférée Q représentera un reste de polyéthylèneglycol (PEG) plutôt qu'un reste de polypropylèneglycol (PPG). Par commodité et soucis d'homogénéité, on a utilisé ci-après les expressions "reste de polyalkylèneglycol" et "reste de polyéthylèneglycol" même quand dans la formule I le nombre n est égal à 1.

Le groupe A représente, comme indiqué ci-dessus, le reste d'un α-monoaminoacide basique. Il provient d'un monoaminoacide ayant un pH supérieur à 7, c'est-à-dire comportant une fonction acide COOH et, en plus de la fonction basique en α, au moins une fonction basique latérale. Parmi les restes d'α-aminoacides qui conviennent, on peut mentionner notamment les restes Dbu, Arg, Lys, His et Orn. Les restes A préférés sont des restes d'α-L-aminoacides, notamment L-Arg, L-Lys et L-Orn, le reste le plus intéressant selon l'invention étant L-Arg.

Le groupe B représente comme indiqué plus haut une simple liaison, un reste monoaminoacide ou une chaîne peptidique renfermant de 2 à 9 aminoacides. Les aminoacides qui interviennent dans le groupe B peuvent être quelconques, notamment naturels ou synthétiques. En particulier, quand B représente un reste de monoaminoacide, ledit reste sera soit un reste dépourvu d'atome de carbone asymétrique (tel que notamment Aib, Cle, Gly, MeGly et 4Abu) soit un reste comportant un atome de carbone asymétrique et étant de configuration L ou D, le ou les autres restes aminoacides de la chaîne peptidique étant chacun un aminoacide dépourvu d'atome de carbone asymétrique ou un aminoacide comportant un atome de carbone asymétrique et étant de configuration L.

Lorsque B est un reste de monoaminoacide, il provient d'un aminoacide naturel ou synthétique ayant un pH inférieur ou égal à 7 ou d'un aminoacide ayant un pH supérieur à 7 mais convenablement substitué pour bloquer essentiellement son caractère basique et atteindre un pH inférieur ou égal à 7.

B englobe donc alors :

1) les restes d'aminoacides naturels ou synthétiques qui comprennent une seule fonction basique,

2) les restes d'aminoacides naturels ou synthétiques qui comprennent une seule fonction basique et plus d'une fonction acide et dans lesquels chaque fonction acide latérale (n'entrant pas ou n'intervenant pas dans la liaison peptidique) est susceptible d'être bloquée notamment sous forme amide ou ester, et

3) les restes d'aminoacides naturels ou synthétiques qui comprennent une seule fonction acide et plus d'une fonction basique et dans lesquels chaque fonction basique latérale est convenablement substituée pour bloquer et supprimer essentiellement son caractère basique.

Bien entendu les restes d'aminoacides sus-visés, qui sont notamment énumérés dans le chapitre ABREVIATIONS ci-dessus, peuvent comporter des groupes hydroxyle (OH) ou thiol (SH) latéraux qui sont

susceptibles d'être bloqués, le cas échéant, par un groupe protecteur éther ou ester.

Conviennent en particulier pour B, les restes provenant d'aminoacides :

- naturels non basiques dits "neutres" tels que notamment Ala, β-Ala, Cys, Gln, 4Hyp, 3Hyp, Leu, Met, Nle, Nva, Phe, Pro, MeGly, Ser, Tyr, Val, où le groupe OH latéral des restes 4Hyp, 3Hyp, Ser, Thr, Tyr est protégé ou non par un groupe protecteur éther ou ester, et où le groupe SH latéral du reste Cys est protégé ou non par un groupe protecteur thioéther ou thioester;
- naturels acides tels que notamment Asp, β-Asp, Glu, γ-Glu, dans lesquels la fonction acide latérale est libre ou bloquée sous forme amide ou ester, notamment sous forme d'ester benzylique ou t-butylique;
- naturels initialement basiques dans lesquels la ou les fonctions basiques latérales n'entrant pas dans la liaison peptidique sont bloquées par un groupe acide convenable éliminant pratiquement le caractère basique tel que Adoc, Aoc, Boc, Cbo, Foc, Fmoc, Iboc, Z, Z(p-Cl) et Z(p-OMe) etc., lesdits restes d'aminoacide étant notamment les restes Arg, Lys, His et Orn convenablement bloqués notamment par un groupe Cbo;
- synthétiques non basiques dits "neutres" tels que notamment ACC, AHX, Aib, ATC, Aze, Abu, 4Abu, CHA, CHG, CHT (où le groupe OH latéral est protégé le cas échéant sous forme éther ou ester), Cle, Phg, Pip, Pyr;
- synthétiques initialement basiques dans lesquels chaque fonction basique latérale est protégée par un groupe convenable éliminant substantiellement le caractère basique, notamment le reste Dbu, où la fonction basique latérale est bloquée par un groupe tel que Cbo.

Parmi les restes d'aminoacides synthétiques qui conviennent également, on peut mentionner (i) les restes d'aminoacides cycloaliphatiques où le groupe amino et le groupe carboxy sont situés sur le même atome de carbone cyclique (tels que le groupe Cle sus-visé) ou sur deux atomes de carbone cycliques différents et (ii) les restes d'aminoacides aromatiques tels que notamment les restes o-, m- et p-aminobenzoyle, et, les restes d'aminoacides aralkyliques tels que notamment les p-aminobenzylcarbonyle, m-aminobenzylcarbonyle.

De façon avantageuse, le reste monoaminoacide de B, selon l'invention, sera un reste d'α-aminoacide tel que ACC, Aib, Ala, ATC, Asp, Aze, But, CHA, CHG, Cle, Glu, Gly, 4Hyp, 3Hyp, Ile, Leu, Met, Nle, Nva, Phe, Pip, Pro, MeGly, Ser, Thr, Tyr, Val, un reste β-aminoacide tel que β-Ala, un reste CHT, 4Hyp, 3Hyp, Ser, Thr, Tyr dans lequel le groupe OH latéral est protégé sous forme éther ou ester, ou un reste Asp ou Glu dans lequel le groupe OH de la fonction acide carboxylique latérale est remplacé par un groupe $OR_3$, $NH_2$, $NHR_4$ ou $NR_4R_5$ où $R_3$ est alkyle en $C_1$-$C_4$ (de préférence Me, Et, iBu, Bu), hydroxyalkyle en $C_1$-$C_4$ - (de préférence $CH_2CH_2OH$), cycloalkyle en $C_3$-$C_6$ (tel que cyclopropyle, cyclopentyle ou cyclohexyle) ou ω-aminoalkyle [où le fragment alkyle est en $C_2$-$C_4$ et où le groupe amino peut être monoalkylé ($NHR_4$), dialkylé ($NR_4R_5$) ou inclus dans un cycle ($NR_4R_5$ = groupe hétérocyclique)], $R_4$ et $R_5$, identiques ou différents, représentent chacun un groupe alkyle en $C_1$-$C_4$, $R_4$ et $R_5$ considérés ensemble pouvant former avec l'atome d'azote, auquel ils sont liés, un groupe N-hétérocyclique choisi parmi l'ensemble constitué par les groupes pyrrolidino, pipéridino, morpholino, thiomorpholino, pipérazino, 4-(2-hydroxyéthyl)pipérazino, 4-méthylpipérazino, 4-(4-chlorophényl)pipérazino et hexaméthylèneimino.

Le reste monoaminoacide B comprend donc des restes d'aminoacides tels que Aib, β-Ala, Cle, Gly, MeGly et 4Abu dépourvus d'atome de carbone asymétrique, et des restes d'aminoacides présentant un atome de carbone asymétrique.

Lorsque l'unique aminoacide présent dans B comporte un atome de carbone asymétrique, le reste dudit aminoacide sera de préférerce de configuration L, car l'expérience montre que les peptides de formule I dans lesquels B est un reste monoaminoacide de configuration D sont en général moins actifs en tant que substrats.

Le groupe monoaminoacide B sera de préférence choisi parmi l'ensemble constitué par

(i) les restes Aib, L-Ala, β-Ala, L-ATC, L-Aze, L-Abu, L-CHA, L-CHG, L-CHT, Cle, Gly, L-4Hyp, L-3Hyp, L-Asp, L-Glu, L-Ile, L-Leu, L-Nle, L-Nva, L-Phe, L-Pip, L-Pro, MeGly, L-Ser, L-Thr, L-Tyr et L-Val,

(ii) les restes L-CHT, L-4Hyp, L-3Hyp, L-Ser, L-Thr et L-Tyr où le groupe OH latéral est protégé sous la forme d'un groupe ester ou éther,

(iii) les restes L-Arg, L-Lys, L-His, L-Orn et L-Dbu où le groupe basique latéral est substitué par un groupe approprié pour éliminer substantiellement le caractère basique dudit groupe latéral, ledit groupe approprié provenant d'un groupe acide, notamment Cbo et

(iv) les restes L-Asp ou L-Glu où le groupe acide carboxylique latéral est estérifié ou amidifié.

Lorsque B est un groupe peptidique renfermant de 2 à 9 restes aminoacides, chacun des aminoacides de la chaîne peptidique sera choisi parmi les restes aminoacides énumérés ci-dessus en ce qui concerne le groupe B = monoaminoacide. De préférence, la chaîne peptidique constituée par B ne comportera dans ce

cas que des restes α-aminoacides.

De façon également préférée, l'aminoacide dont l'extrémité N-terminale de ladite chaîne peptidique, liée au groupe Q par l'intermédiaire d'un pont carbonyle, sera un reste α-aminoacide choisi parmi l'ensemble constitué par

(i) les restes α-aminoacides dépourvus d'atome de carbone asymétrique, tels que Aib, Cle et Gly, et

(ii) les restes α-aminoacides présentant un atome de carbone asymétrique, et étant de configuration D ou L,

le ou les autres restes aminoacides de ladite chaîne peptidique étant de préférence (a) Aib, Cle, Gly ou (b) un ou des restes d'α-aminoacide de configuration L.

De plus, de façon avantageuse, lorsque B représentera une chaîne peptidique, ladite chaîne peptidique comportera 2 ou 3 aminoacides, c'est-à-dire que les composés de formule I seront alors des bis-(tripeptides) ou des bis(tétrapeptides).

Par ailleurs, quand B est une chaîne peptidique comportant 2 à 3 restes aminoacides, on recommande en particulier que le reste aminoacide en position 2, c'est-à-dire celui qui est directement lié au reste aminoacide comportant l'extrémité N-terminale de B-A, soit Asp ou Glu, le groupe carboxy latéral de chaque reste Asp ou Glu pouvant, le cas échéant, être estérifié ou amidifié, comme indiqué ci-dessus, pour former des composés de formule I particulièrement utiles dans le domaine de la détermination du facteur Xa intervenant dans l'hémostase.

Le moyen de marquage R est bien connu dans la technique des dosages biologiques et microbiologiques, voir à cet effet l'art antérieur précité et notamment le document US-A-4 448 715. Ledit moyen de marquage sera de préférence choisi parmi l'ensemble des groupes aminés NH-R' qui (i) induisent une modification de coloration, (ii) induisent une modification de fluorescence ou (iii) comportent au moins un élément radioactif (par exemple un groupe anilino ou benzylamino marqué par un radioisotope $^{14}C$ ou $^{3}H$). Convient au but de l'invention tout groupe amino NH-R' donnant pendant ou après la réaction enzymatique un signal susceptible d'être amplifié pour être détecté (par exemple par mesure de la densité optique sous une longueur d'onde donnée, ou encore par mesure de la radioactivité). La quantité de produit H-R obtenu par clivage lors de l'hydrolyse enzymatique est proportionnelle à la quantité d'enzyme utilisée. Ladite quantité de H-R est déterminable photométriquement, spectrophotométriquement, fluorospectrophotométriquement ou électrochimiquement.

Le groupe R que l'on recommande suivant l'invention est un groupe chromogène, de façon typique un groupe nitrophénylamino (dans lequel le reste phényle est susceptible d'être substitué par un groupe COOH, F, Cl, Br, $CH_3$, $OCH_3$, CN, $CF_3$, et/ou $SO_3H$), ou un groupe fluorogène, de façon typique un groupe naphtylamino (dans lequel le reste naphtyle est susceptible d'être substitué par un groupe $OCH_3$, COOH, $SO_3H$ ou $CH_3$), et les groupes 4-méthylcoumaryl-7-amino, 4-trifluorométhylcoumaryl-7-amino et analogues.

Parmi les groupes aminés chromogènes et fluorogènes, qui conviennent suivant l'invention, on peut notamment citer les groupes p-nitroanilino (en abrégé pNA), 2-carboxy-4-nitroanilino et 3-carboxy-4-nitroanilino, 2-halogéno-4-nitroanilino et 3-halogéno-4-nitroanilino (où l'halogène est F, Cl ou Br), 2-méthoxy-5-méthyl-4-nitroanilino, 2-hydroxysulfonyl-4-nitroanilino, 4-trifluorométhyl-2-nitroanilino, 4-trifluorométhyl-3-nitroanilino, 4-cyano-2-nitroanilino, 2-naphtylamino, 4-hydroxysulfonyl-1-naphtylamino, quinolylamino, nitroquinolylamino et analogues.

Le groupe R préféré suivant l'invention est un groupe chromogène à savoir, d'une part, pNA qui convient d'une manière générale à la détermination quantitative des enzymes protéolytiques sus-visées et est particulièrement adapté à la détermination du Facteur Xa, de la protéine C et/ou de la plasmine, et d'autre part, les groupes analogues, où le noyau phényle de pNA est substitué en position 2 ou 3, et répondant à la formule

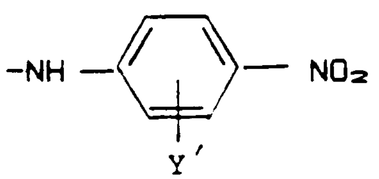

(II)

dans laquelle Y' est Br, Cl, F, $CF_3$, COOH, COOW, $CONH_2$, CONHW, $CONW_2$, $CONH(CH_2)_mNMe_2$, OH ou OW, où W est un groupe alkyle en $C_3$-$C_6$, aryle en $C_6$-$C_{10}$, aralkyle en $C_7$-$C_{11}$ ou alicyclique en $C_3$-$C_8$ et m est un nombre entier ayant pour valeur 1 à 10.

De tels groupes de formule II où le noyau phényle du groupe pNA est substitué sont notamment décrits dans le document EP-A-0 110 306.

Les sels d'addition suivant l'invention sont essentiellement des sels d'addition d'acide obtenus par réaction d'un composé de formule I avec un acide minéral ou organique.

Le meilleur mode de mise en oeuvre de l'invention consiste à utiliser comme substrat un composé bipeptide symétrique choisi parmi l'ensemble constitué par

(i) les composés de formule

pNA-Arg-B-CO-O[$CH_2CH_2O$]$_n$-CO-B-Arg-pNA (Ia)

dans laquelle

B est une simple liaison; un reste Aib, Cle, Gly ou un reste de monoaminoacide de configuration L; ou, un reste di- ou tripeptidique dans lequel le reste aminoacide de l'extrémité N-terminale de la chaîne peptidique qui est liée au pont carbonyle CO, est Aib, Cle, Gly ou de configuration L ou D, le ou les autres restes aminoacides étant Aib, Cle, Gly ou de configuration L;

n est un nombre entier supérieur ou égal à 1 tel que le poids moléculaire moyen du groupe O-[$CH_2CH_2O$]$_n$ soit compris entre environ 60 et environ 600 et mieux entre 200 et 400; et,

(ii) leurs sels d'addition.

D'un point de vue pratique, dans le cadre du meilleur mode de mise en oeuvre de l'invention, il est particulièrement avantageux que le poids moléculaire moyen du groupe O[$CH_2CH_2O$]$_n$ soit de l'ordre de 200-400, c'est-à-dire que le groupe O[$CH_2CH_2O$]$_n$ provienne d'un composé PEG ayant une masse moléculaire de 200-400, d'une part, et que le groupe B soit un reste di- ou tripeptidique, d'autre part.

De façon non limitative, on a consigné dans le tableau I ci-après un certain nombre de composés peptidiques selon l'invention, les plus intéressants étant en particulier :

(a) $PEG_{200}$ (CO-Gly-L-Pro-L-Arg-pNA)$_2$
(b) $PEG_{600}$ (CO-Gly-L-Pro-L-Arg-pNA)$_2$
(c) $PEG_{200}$ (CO-D-Glu(OBzl)-L-Pro-L-Arg-pNA)$_2$
(d) $PEG_{200}$ (CO-D-Nle-L-CHA-L-Arg-pNA)$_2$
(e) $PEG_{200}$ (CO-D-Leu-Gly-L-Arg-pNA)$_2$
(f) $PEG_{200}$ (CO-D-CHA-Gly-L-Arg-pNA)$_2$
(g) $PEG_{300}$ (CO-D-Leu-Gly-L-Arg-pNA)$_2$
(h) $PEG_{400}$ (CO-D-Leu-Gly-L-Arg-pNA)$_2$
(i) $PEG_{200}$ (CO-D-Nle-Gly-L-Arg-pNA)$_2$, et
(j) leurs sels d'addition.

Comme les substrats antérieurement connus, les composés symétriques de formule I ou Ia et leurs sels d'addition selon l'invention, donnent par hydrolyse enzymatique une quantité de H-R proportionnelle à la quantité d'enzyme présente dans l'échantillon à tester. En revanche, par rapport auxdits substrats antérieurement connus, les composés selon l'invention présentent une cinétique d'hydrolyse enzymatique différente.

**TABLEAU I**

**pNA-A-B-CO-Q-CO-B-A-pNA, xHAo**

| Produit | Q | B(1) | A | x HAo |
|---|---|---|---|---|
| Ex 1 | $PEG_{200}$ | Gly-L-Pro | L-Arg | 2H-TFA |
| Ex 2 | $PEG_{200}$ | Gly-L-Pro | L-Arg | 2AcOH |
| Ex 3 | $PEG_{600}$ | Gly-L-Pro | L-Arg | 2A‹OH |
| Ex 4 | $PEG_{200}$ | D-Glu(OBzl)-L-Pro | L-Arg | 2AcOH |
| Ex 5 | $PEG_{200}$ | D-Nle-L-CHA | L-Arg | 2AcOH |
| Ex 6 | $PEG_{200}$ | D-Leu-Gly | L-Arg | 2AcOH |
| Ex 7 | $PEG_{200}$ | D-CHA-Gly | L-Arg | 2AcOH |
| Ex 8 | $PEG_{300}$ | D-Leu-Gly | L-Arg | 2AcOH |
| Ex 9 | $PEG_{400}$ | D-Leu-Gly | L-Arg | 2AcOH |
| Ex 10 | $PEG_{200}$ | D-Nle-Gly | L-Arg | 2AcOH |
| Ex 11 | $PEG_{400}$ | Gly-L-Pro | L-Arg | 2AcOH |
| Ex 12 | $PEG_{200}$ | D-CHA-L-3Hyp | L-Arg | 2AcOH |
| Ex 13 | $PEG_{200}$ | Gly-L-Pip | L-Arg | 2AcOH |
| Ex 14 | $PEG_{400}$ | Gly-L-Pip | L-Arg | 2AcOH |
| Ex 15 | $PEG_{200}$ | D-Lys(ε-Cbo)-L-ATC | L-Arg | 2AcOH |
| Ex 16 | $PEG_{400}$ | D-CHA-L-Nle | L-Arg | 2AcOH |
| Ex 17 | $PEG_{200}$ | D-Ala-L-Nva | L-Arg | 2AcOH |
| Ex 18 | $PEG_{200}$ | D-THr(Bzl)-AHX | L-Lys | 2AcOH |
| Ex 19 | $PEG_{400}$ | D-Pro-L-Pro | L-Arg | 2AcOH |
| Ex 20 | $PEG_{200}$ | D-Lys(ε-Cbo)-L-Phe | L-Arg | 2AcOH |

**TABLEAU I (suite 1)**

| Produit | Q | B(1) | A | x HAo |
|---|---|---|---|---|
| Ex 21 | $PEG_{400}$ | Gly-L-Phe | L-His | 2AcOH |
| Ex 22 | $PEG_{200}$ | D-But-L-Leu | L-Arg | 2AcOH |
| Ex 23 | $PEG_{200}$ | D-Lys(ε-Cbo)-L-Leu | L-Arg | 2AcOH |
| Ex 24 | $PEG_{400}$ | D-Ser(Bzl)-L-Leu | L-Arg | 2AcOH |
| Ex 25 | $PEG_{200}$ | D-Ala-L-4Hyp | L-Arg | 2AcOH |
| Ex 26 | $PEG_{200}$ | D-CHG-L-Abu | L-Arg | 2AcOH |
| Ex 27 | $PEG_{400}$ | D-Leu-L-Pro | L-Arg | 2AcOH |
| Ex 28 | $PEG_{400}$ | D-But-L-CHA | L-Arg | 2AcOH |
| Ex 29 | $PEG_{200}$ | D-Lys(ε-Cbo)-L-Pro | L-Arg | 2AcOH |
| Ex 30 | $PEG_{200}$ | D-Nle-L-CHA | L-Lys | 2AcOH |
| Ex 31 | $PEG_{400}$ | D-Pro-L-Pro | L-Orn | 2AcOH |
| Ex 32 | $PEG_{400}$ | D-Leu-Gly | L-Orn | 2AcOH |
| Ex 33 | $PEG_{400}$ | D-Glu-Gly-L-Leu | L-Arg | 2AcOH |
| Ex 34 | $PEG_{200}$ | D-Leu-L-Leu-L-CHA | L-Arg | 2AcOH |
| Ex 35 | $PEG_{200}$ | D-Thr(Bzl)-L-Leu-L-CHA | L-Arg | 2AcOH |
| Ex 36 | $PEG_{400}$ | D-CHA-Gly | L-Arg | 2AcOH |
| Ex 37 | $PEG_{400}$ | D-Nle-Gly | L-Arg | 2AcOH |

**TABLEAU I** (suite 2)

| Produit | Q | B(1) | A | x HAo |
|---|---|---|---|---|
| Ex 38 | $PEG_{400}$ | D-CHG-Gly | L-Arg | 2AcOH |
| Ex 39 | $PEG_{600}$ | D-CHA-Gly | L-Arg | 2AcOH |
| Ex 40 | $PEG_{400}$ | D-CHT-Gly | L-Arg | 2AcOH |
| Ex 41 | $PEG_{400}$ | D-Nva-Gly | L-Arg | 2AcOH |
| Ex 42 | $PEG_{400}$ | D-Val-Gly | L-Arg | 2AcOH |
| Ex 43 | $PEG_{400}$ | D-Ile-L-Glu-Gly | L-Arg | 2AcOH |
| Ex 44 | $PEG_{400}$ | D-Ile-L-Glu(OMe)-Gly | L-Arg | 2AcOH |
| Ex 45 | $PEG_{400}$ | D-Ile-L-Glu(Py)-Gly | L-Arg | 2AcOH |
| Ex 46 | $PEG_{400}$ | D-Ile-L-Asp(Mor)-Gly | L-Arg | 2AcOH |
| Ex 47 | $PEG_{400}$ | D-Phe-Gly | L-Arg | 2AcOH |
| Ex 48 | $PEG_{400}$ | D-Lys(ε-Cbo)-Gly | L-Arg | 2AcOH |
| Ex 49 | $PEG_{400}$ | D-CHG-L-Glu(OMe)-Gly | L-Arg | 2AcOH |
| Ex 50 | $PEG_{400}$ | D-Leu-MeGly | L-Arg | 2AcOH |
| Ex 51 | $PEG_{400}$ | D-Nle-L-Glu(OiPr)-Gly | L-Arg | 2AcOH |
| Ex 52 | $PEG_{400}$ | D-Ile-L-Glu(OtBu)-Gly | L-Arg | 2AcOH |
| Ex 53 | $PEG_{200}$ | D-CHG-Aib-L-CHA | L-Arg | 2AcOH |
| Ex 54 | $PEG_{400}$ | D-Leu-Cle-L-Pro | L-Arg | 2AcOH |
| Ex 55 | $PEG_{400}$ | D-Pro-Cle-L-Leu | L-Arg | 2AcOH |
| Ex 56 | $PEG_{400}$ | D-Nle-Aib-L-Pro | L-Arg | 2AcOH |
| Ex 57 | $PEG_{400}$ | D-Nva-Gly-L-Pro | L-His | 2AcOH |

**TABLEAU I (fin)**

| Produit | Q | B(1) | A | x HAo |
|---|---|---|---|---|
| Ex 58 | PEG200 | Aib-L-CHG-L-Pro | L-Arg | 2AcOH |
| Ex 59 | PEG200 | Aib-L-Lys(ε-Cbo)-L-Pro | L-Arg | 2AcOH |
| Ex 60 | PEG200 | Gly-L-Leu-L-CHA | L-Arg | 2AcOH |
| Ex 61 | PEG400 | Gly-L-Thr(Bzl)-L-Abu | L-Arg | 2AcOH |
| Ex 62 | PEG400 | D-Leu-L-Asp(Pi)-Gly | L-Arg | 2AcOH |
| Ex 63 | PEG200 | D-Val-L-Glu(Pi)-Gly | L-Arg | 2AcOH |
| Ex 64 | PEG60 | D-Leu-Gly | L-Arg | 2AcOH |
| Ex 65 | PEG60 | D-Nle-Gly | L-Arg | 2AcOH |
| Ex 66 | PEG100 | D-Leu-Gly | L-Arg | 2AcOH |
| Ex 67 | PEG150 | D-Leu-Gly | L-Arg | 2AcOH |

Notes

(1) Le premier aminoacide cité est lié par son extrémité N-terminale au pont carbonyle CO.

Produits de comparaison :

CP 1 : $MeSO_2$-D-Leu-Gly-L-Arg-pNA, AcOH
CP 2 : H-D-CHG-L-Abu-L-Arg-pNA, 2AcOH
CP 3 : H-D-Abu-L-CHA-L-Arg-pNA, 2AcOH

Les composés de formule I selon l'invention peuvent être préparés selon une méthode connue en soi par application de mécanismes réactionnels classiques.

Le procédé que l'on préconise ici est caractérisé en ce qu'il consiste à faire réagir un composé aminoacide ou peptidique de formule :

H-B-A-R (IV)

où B, A et R sont définis comme indiqué ci-dessus, avec un dihalogénoformiate de polyalkylèneglycol de formule :

Hal-CO-Q-CO-Hal (V)

dans laquelle Hal représente un atome d'halogène, notamment F, Cl, Br et, de préférence, Cl, et, Q est

défini comme indiqué ci-dessus, suivant la réaction :

2(H-B-A-R) + Hal-CO-Q-CO-Hal ---> I

Les composés de départ de formules IV et V sont obtenus selon des méthodes classiques bien connues de l'homme de métier. En particulier, le composé IV, quand B est différent d'une simple liaison, est obtenu selon une des méthodes usuelles de la synthèse peptidique.

De façon avantageuse, on fera réagir 2 moles de IV avec 1 à 1,3 moles (de préférence 1 à 1,1 moles) de V dans un solvant inerte (notamment DMF ou THF) à une température comprise entre 0 et 40°C pendant au moins 0,25 h, en présence d'un moyen accepteur de protons, notamment une amine tertiaire telle que $Et_3N$ ou DIEA intervenant également en tant que co-solvant du milieu réactionnel. De préférence, on utilisera un excès d'amine tertiaire par rapport aux conditions stoechiométriques, notamment selon un rapport molaire amine tertiaire/composé IV de 1,7/1 à 2,6/1 et mieux de 2/1 à 3,1/1.

Les composés tripeptides de formule I et leurs sels d'addition sont utiles dans la détermination des enzymes intervenant dans l'hémostase. Ils constituent notamment des substrats spécifiques du Facteur Xa de la plasmine ou de la thrombine. Le procédé de détermination de ces enzymes que l'on préconise est caractérisé en ce que l'on met en contact dans un milieu liquide aqueux approprié tel qu'un sérum physiologique tamponné, une quantité donnée d'un peptide de formule I ou l'un de ses sels d'addition (à une concentration de l'ordre de 10 mg/ml) et un échantillon à tester (éventuellement dilué) susceptible de contenir l'enzyme cible, pendant au moins 0,5 h à une température compris entre RT et 40°C, notamment 37°C.

L'activité des peptides suivant l'invention vis-à-vis d'enzymes intervenant au niveau de l'hémostase a été déterminée selon des méthodes classiques, telles que décrites dans EP-A-0 280 610 (voir page 14), en appréciant la vitesse d'hydrolyse par la variation de la densité optique dans le temps ($\Delta$OD/min). Les résultats qui ont été obtenus ont été consignés dans le tableau II ci-après où par commodité, l'activité de produits de référence (CP 1 à CP 3) est précisée comme étant égale à 100 % .

D'autres avantages et caractéristiques de l'invention seront mieux compris à la lecture qui va suivre d'exemples de préparation et de résultats d'essais comparatifs. Bien entendu, l'ensemble de ces éléments n'est nullement limitatif mais est donné à titre d'illustration.

**PREPARATION I**

Obtention de $PEG_{200}$ (D-Leu-Gly-L-Arg-pNA)$_2$, 2AcOH (Exemple 5)

a) Z-L-Arg-pNA, HCl

On dissout 344 g (1 mole) de Z-L-Arg-OH, HCl dans du HMPT anhydre (fraichement distillé et séché sur tamis moléculaire) à RT, puis on ajoute 139 ml (1 mole) de $Et_3N$ à RT sous agitation. A la solution résultante, on ajoute 328 g (2 moles) de p-nitrophénylisocyanate. Le milieu réactionnel résultant est maintenu sous agitation pendant 24 h à RT, puis est évaporé sous vide et repris avec une quantité minimale de AcOH avant d'être dilué avec AcOEt. La solution résultante est ensuite extraite successivement trois fois avec de petites quantités de $NaHCO_3$ 0,5 M, trois fois avec une solution de $KHSO_4$ à 50 g/l puis plusieurs fois avec $H_2O$ à demi-saturée en NaCl. La phase organique est ensuite séchée sur sulfate de sodium anhydre. Après filtration (élimination de $Na_2SO_4$), on évapore le solvant et recristallise le résidu d'évaporation dans le mélange AcOEt/MeOMe (3/7) v/v. On obtient 350 g du produit attendu sous la forme d'une poudre de couleur blanche.

F = 128-130°C.

Analyse (CCM sur gel de silice) :

Rf = 0,5 dans AcOEt/pyridine/AcOH/$H_2O$ (20/4,5/3/1) v/v;

Rf = 0,69 dans $CHCl_3$/MeOH/AcOH (5/3/1) v/v.

b) H-L-Arg-pNA, 2HBr

Dans un appareillage en verre et téflon, on charge 100 g (0,215 moles) de Z-L-Arg-pNA, HCl. On ajoute, sous atmosphère inerte (courant d'azote), successivement 800 ml de AcOH glacial, 200 ml d'anisol, 1000 ml de HBr en solution dans AcOH glacial. On laisse réagir pendant 1 h à RT sous atmosphère d'azote. Après ce laps de temps, le mélange réactionnel, qui est devenu homogène au cours de la déprotection, est précipité dans 20 l d'éther (MeOMe ou EtOEt). Après décantation, le surnageant est écarté et le précipité

est lavé plusieurs fois à l'éther. On recueille le précipité par filtration, le sèche sous vide sur KOH pendant 24 h. On obtient 94,2 g (rendement : 96 %) du produit attendu .

Analyse (CCM sur gel de silice) :

Rf = 0,04 dans AcOEt/pyridine/AcOH/$H_2O$ (20/4,5/3/1,5) v/v

Rf = 0,38 dans BuOH/AcOH/$H_2O$ (3/1/1) v/v.

c) Boc-Gly-L-Arg-pNA, HBr

On dissout 1 g (2,19 mmoles) de H-L-Arg-pNA, 2HBr dans 10 ml de DMF puis on ajoute 0,854 ml (6,57 mmoles) de DIEA. Dans un autre récipient, on neutralise 384 mg (2,19 mmoles) de Boc-Gly-OH dissous dans 5 ml de DMF au moyen d'une quantité de 0,285 ml de DIEA. On mélange les deux solutions ainsi obtenues et au milieu résultant, on ajoute 970 mg de Bop tout en maintenant la température à RT; on maintient également le pH à une valeur comprise entre 7 et 8 par ajout de petites portions de DIEA au cours de la réaction. Au bout d'une heure, la réaction est complètement terminée et on évapore à sec sous vide le milieu réactionnel; on reprend le résidu d'évaporation par un mélange AcOEt/MeOH et extrait au moyen d'une solution aqueuse de $NaHCO_3$ 0,5 M. On sèche la phase organique sur sulfate de sodium, la concentre sous vide puis précipite dans l'éther (MeOMe ou EtOEt). On obtient 874 mg (rendement : 75 %) de produit attendu.

Analyse (CCM sur gel de silice) :

Rf = 0,53 dans $CHCl_3$/MeOH/AcOH (10/3/1) v/v.

d) H-Gly-L-Arg-pNA, 2H-TFA

On charge dans un réacteur 874 mg (1,64 mmoles) de Boc-Gly-L-Arg-pNA, HBr puis ajoute successivement 6,6 ml de $CH_2Cl_2$ et 6,6 ml de H-TFA. Après 0,25 h de réaction à RT, on précipite directement le mélange réactionnel dans l'éther. Il se forme un précipité blanc floconneux que l'on filtre et sèche. On obtient 910 mg (rendement : 96 %) de produit attendu.

Analyse (CCM sur gel de silice) :

Rf = 0,09 dans $CHCl_3$/MeOH/AcOH (5/3/1) v/v.

e) Boc-D-Leu-Gly-L-Arg-pNA, H-TFA

En suivant les modalités opératoires de la préparation Ic, on fait réagir un mélange réactionnel comprenant (i) 910 mg (1,57 mmoles) de H-Gly-L-Arg-pNA, 2H-TFA, (ii) 363 mg (1,57 mmoles) de Boc-D-Leu-OH, (iii) 1,2 ml de DIEA et (iv) 695 mg (1,57 mmoles) de Bop dans le DMF, et on maintient le mélange réactionnel à RT sous agitation pendant 2 h. Après évaporation à sec sous vide, le résidu d'évaporation est chromatographié sur gel de silice avec comme éluant un gradient de $CHCl_3$/MeOH/AcOH de (20/3/1) à (10/3/1) v/v. On réunit les fractions homogènes recueillies et évapore l'éluant. Par lyophilisation, on obtient 785 mg (rendement : 80 %) de produit pur attendu.

Analyse (CCM sur gel de silice) :

Rf = 0,33 dans $CHCl_3$/MeOH/AcOH (10/3/1) v/v.

f) H-D-Leu-Gly-L-Arg-pNA, 2H-TFA

On fait réagir 785 mg (1,25 mmoles) de Boc-D-Leu-Gly-L-Arg-pNA, H-TFA obtenu selon la préparation Ie avec 5 ml de $CH_2Cl_2$ et 5 ml de H-TFA à RT. Au bout de 0,25 h, le mélange réactionnel est directement précipité dans l'éther. On obtient un précipité blanc que l'on filtre, lave à l'éther et sèche. On recueille 779 mg (rendement : 90 %) du produit attendu.

Analyse (CCM sur gel de silice) :

Rf = 0,2 dans $CHCl_3$/MeOH/AcOH (5/3/1) v/v.

g) $PEG_{200}$(D-Leu-Gly-L-Arg-pNA)$_2$, 2AcOH

A 779 mg (1,12 mmoles) de H-D-Leu-Gly-L-Arg-pNA, 2H-TFA, obtenu comme indiqué ci-dessus, on ajoute 15 ml de DMF et 0,345 ml (2,47 mmoles) de $Et_3N$. Le mélange résultant est refroidi dans de la glace sous agitation. On ajoute ensuite goutte à goutte 182 mg (0,563 mmole) de dichloroformiate de $PEG_{200}$ - [obtenu selon la préparation VIII ci-après et répondant à la formule développée : Cl-CO-O($CH_2CH_2O$)$_n$-CO-Cl où n est tel que le PM du fragment O($CH_2CH_2O$)$_n$ ait une valeur de 200 environ] dans 5 ml de DMF. On

laisse ensuite le mélange réactionnel résultant se réchauffer lentement jusqu'à RT en 2 h. On filtre pour éliminer le sel de triéthylammonium qui s'est formé et on évapore le filtrat. Le résidu d'évaporation est chromatographié sur une résine échangeuse d'ions (AMBERLITE[R] IRA 401 S) avec comme éluant un mélange MeOH/$H_2O$ (3/2) v/v. On réunit les fractions homogènes obtenues. Par évaporation puis lyophilisation, on obtient 460 mg (rendement : 63 %) du produit attendu.

Analyse (CCM sur gel de silice) :
Rf = 0,34 dans $CHCl_3$/MeOH/AcOH (5/3/1) v/v.

**PREPARATION II**

Obtention de $PEG_{300}$(D-Leu-Gly-L-Arg-pNA)$_2$, 2AcOH (Exemple 8)

En suivant le mode opératoire donné à la préparation Ig et en remplaçant le dichloroformiate de $PEG_{200}$ par le dichloroformiate de $PEG_{300}$, on obtient le produit attendu.

**PREPARATION III**

Obtention de $PEG_{400}$(D-Leu-Gly-L-Arg-pNA)$_2$, 2AcOH (Exemple 9)

En suivant le mode opératoire donné à la préparation Ig et en remplaçant le dichloroformiate de $PEG_{200}$ par le dichloroformiate de $PEG_{400}$, on obtient le produit attendu.

**PREPARATION IV**

Obtention de $PEG_{200}$(D-Nle-Gly-L-Arg-pNA)$_2$, 2AcOH (Exemple 10)

En suivant le mode opératoire donné à la préparation I mais en remplaçant au stade Ie l'aminoacide de formule Boc-D-Leu-OH par l'aminoacide de formule Boc-D-Nle-OH on obtient le produit attendu.

**PREPARATION V**

Obtention de $PEG_{200}$(D-Nle-L-CHA-L-Arg-pNA)$_2$, 2AcOH (Exemple 5)

En suivant le mode opératoire donné à la préparation I mais en remplaçant au stade Ic le Boc-Gly-OH par le Boc-L-CHA-OH et au stade Ie le Boc-D-Leu-OH par l'aminoacide de formule Boc-D-Nle-OH, on obtient le produit attendu.

**PREPARATION VI**

Obtention de $PEG_{200}$(D-Gly-L-Pro-L-Arg-pNA), 2AcOH (Exemple 2)

a) Z-L-Arg-pNA, HCl

On dissout 100 g (0,724 mole) de H-pNA dans 1,5 l de pyridine. Le mélange sous agitation est refroidi jusqu'à -20 °C. On ajoute goutte à goutte 64 ml (0,724 mole) de $PCl_3$ préalablement refroidi à -20 °C et maintient le mélange réactionnel à -20 °C pendant 1 h. On ajoute ensuite 223 g de Z-L-Arg-OH en maintenant la température à -20 °C pendant 1 h puis on laisse le mélange réactionnel se réchauffer jusqu'à la température ambiante que l'on maintient pendant 12 h. Le mélange réactionnel ainsi obtenu est transféré dans 10 litres d'eau glacée, le précipité surnageant est filtré puis repris par un mélange AcOH/AcOEt et lavé successivement (3 fois) par une solution aqueuse de $NaHCO_3$ 0,5 M, une solution aqueuse de $KHSO_4$ à 50 g/l puis de l'eau à demi-saturée en NaCl. La phase organique est séchée sur $Na_2SO_4$ puis évaporée et concentrée. Le concentrat ainsi obtenu est chromatographié sur une colonne de silice au moyen d'un système gradient $CHCl_3$/MeOH/AcOH de (20/3/1) à (5/3/1) v/v. Les fractions homogènes sont réunies, évaporées et précipitées dans l'éther. On obtient 222 g (rendement : 66 %) du produit attendu. F = 128-130 °C

Analyse (CCM sur gel de silice) :
Rf = 0,76 dans $CHCl_3$/MeOH/AcOH (5/3/1) v/v;
Rf = 0,2 dans $CHCl_3$/MeOH/AcOH (20/3/1) v/v.

b) H-L-Arg-pNA, 2HBr

On procède comme indiqué à la préparation Ib.

c) Boc-Gly-L-Pro-OH

A 1,15 g (10 mmoles) de proline dans 50 ml de DMF on ajoute 1,4 ml (11 mmoles) de N-méthylmorpholine. On refroidit le mélange au moyen d'un bain de glace puis ajoute 3,06 g (10 mmoles) de Boc-Gly-OSu et ajuste pendant la durée de la réaction (14 h) le pH entre 6 et 7 par additions successives de petites quantités de N-méthylmorpholine (cette substance intervient comme accepteur de protons). On évapore le DMF et reprend le résidu d'évaporation par AcOEt; par précipitation dans l'éther de pétrole puis filtration, on recueille le produit attendu.

Analyse (CCM sur gel de silice) :
Rf = 0,53 dans BuOH/AcOH/$H_2O$ (3/1/1) v/v;
Rf = 0,42 dans AcOEt/pyridine/AcOH/$H_2O$ (20/4,5/3/1,5) v/v.

d) Boc-Gly-L-Pro-L-Arg-pNA, HBr

A 4 g (8,8 mmoles) de H-L-Arg-pNA, 2HBr dans 25 ml de DMF, on ajoute 1,35 ml de $Et_3N$. On refroidit au moyen d'un bain de glace puis ajoute successivement 2,38 g de dipeptide Boc-Gly-L-Pro-OH, 2,67 g (17,5 mmoles) de HOBT, 2,7 g (environ 3,15 mmoles) de DCCI, le pH étant ajusté entre 6 et 7 par addition de $Et_3N$. On maintient le milieu réactionnel sous agitation à RT pendant 12 h. Le milieu réactionnel est ensuite filtré (pour écarter la DCHU formée) et le filtrat est évaporé sous vide. Par précipitation au moyen de EtOEt, on obtient un précipité blanc que l'on reprend au moyen d'une quantité minimale de $CHCl_3$/MeOH/AcOH (8/3/1) v/v. On chromatographie la solution résultante sur gel de silice au moyen du même système solvant. On récupère et évapore les fractions homogènes obtenues et par lyophilisation obtient 4,7 g (rendement : 85 %) du produit attendu.

Analyse (CCM sur gel de silice) :
Rf = 0,64 dans BuOH/AcOH/$H_2O$ (3/1/1) v/v;
Rf = 0,76 dans $CHCl_3$/MeOH/AcOH (5/3/1) v/v.

e) H-Gly-L-Pro-L-Arg-pNA, 2H-TFA

En procédant comme indiqué à la préparation If et en remplaçant le Boc-D-Leu-Gly-L-Arg-pNA, H-TFA par 4,7 g de Boc-Gly-L-Pro-L-Arg-pNA, HBr, on obtient après précipitation dans l'éther 5 g du produit attendu.

Analyse (CCM sur gel de silice) :
Rf = 0,12 dans $CHCl_3$/MeOH/AcOH (5/3/1) v/v.

f) $PEG_{200}$(Gly-L-Pro-L-Arg-pNA)$_2$, 2AcOH

A partir du H-Gly-L-Pro-L-Arg-pNA, 2H-TFA, on obtient selon les modalités opératoires décrites à la préparation If le produit attendu.

**PREPARATION VII**

Obtention de $PEG_{200}$(D-Glu(OBzl)-L-Pro-L-Arg-pNA)$_2$, 2AcOH
(Exemple 4)

En procédant comme indiqué dans la préparation VI en remplaçant au stade VIc le Boc-Gly-OSu par le Boc-D-Glu(OBzl)-OSu, on obtient le produit attendu.

**PREPARATION VIII**

Obtention du dichloroformiate de $PEG_{200}$

Dans un réacteur maintenu à -30°C contenant du phosgène, on introduit goutte à goutte au moyen d'une ampoule de coulée 1 g (5 mmoles) de $PEG_{200}$ et 1,4 ml (10 mmoles) de $Et_3N$ dissous dans du THF,

de façon que le phosgène soit toujours dans un large excès par rapport au diol. On laisse le mélange sous agitation pendant 2 h à -30 °C puis on laisse réchauffer jusqu'à RT. L'excès de phosgène est balayé totalement par un courant d'azote pendant plusieurs heures pour éliminer ledit phosgène. Le mélange réactionnel résultant est ensuite évaporé sous vide. On obtient 1,59 g (rendement : 98 %) du produit attendu sous la forme d'un liquide visqueux.

Analyse (spectre IR) :

- disparition des bandes alcool à 3300 $cm^{-1}$;
- apparition des bandes chloroformiate à 1776 et 1152 $cm^{-1}$

On obtient également selon ce procédé les dichloroformiates des autres PEG.

## ESSAIS COMPARATIFS

Le tableau II ci-après résume les résultats des essais comparatifs qui ont été entrepris à des doses équimolaires avec les produits de l'invention et des substances étalons dont l'activité a été prise comme étant égale à 100 % .

TABLEAU II

| ACTIVITES | | | |
|---|---|---|---|
| Produit | Xa | Thrombine | Plasmine |
| Ex 2 | 6 % | 34 % | 130 % |
| Ex 3 | 10 % | 50 % | 122 % |
| Ex 4 | 11 % | 45 % | 289 % |
| Ex 5 | 20 % | 110 % | 15 % |
| Ex 6 | 105 % | 23 % | 188 % |
| Ex 7 | 45 % | 21 % | 112 % |
| Ex 8 | 60 % | 25 % | 150 % |
| Ex 9 | 74 % | 34 % | 165 % |
| CP 1 | 100 % | 18 % | 30 % |
| CP 2 | 14 % | 100 % | 77 % |
| CP 3 | 14 % | 10 % | 100 % |

Lesdits résultats mettent en évidence que, vis-à-vis de la plasmine, les composés selon l'invention Ex 2- Ex 4 et Ex 6- Ex 9 sont plus sensibles que le substrat de référence CP 3 de ladite plasmine, et que le produit Ex 5 est plus sensible que le substrat de référence CP 1 vis-à-vis de la thrombine. Par ailleurs, le produit de l'exemple 6 est utilisable pour la détermination du Facteur Xa quand un inhibiteur de la plasmine est introduit dans l'échantillon à tester.

On préconise enfin selon l'invention, un nécessaire, kit ou trousse de dosage destiné à la détermination d'enzymes intervenant au niveau de l'hémostase, notamment la plasmine, qui est caractérisé en ce qu'il renferme au moins un peptide choisi parmi l'ensemble constitué par les composés de formule I et leurs sels d'addition selon l'invention, et le cas échéant, des échantillons étalons d'enzymes et de milieux tamponnés de dilution.

## Revendications

**1.** Composé appartenant à la famille des aminoacides et des peptides comportant un reste d'aminoacide lié à un reste de polyalkylèneglycol, ledit composé étant caractérisé en ce qu'il est choisi parmi l'ensemble constitué par :

(i) les aminoacides et peptides symétriques de formule :

R-A-B-CO-Q-CO-B-A-R (I)

dans laquelle

Q est un reste de polyalkylèneglycol choisi parmi les restes de polyéthylèneglycol et polypropylèneglycol de formule :

$-O[CH(X)CH_2O]_n-$

où X est H ou Me, et n est un nombre entier supérieur ou égal à 1,

B représente une simple liaison, un reste de monoaminoacide ou une chaîne peptidique comprenant de 2 à 9 restes monoaminoacides,

A est un reste de monoaminoacide basique choisi parmi l'ensemble comprenant les restes d'α-aminoacides comportant un groupe basique sur une chaîne latérale,

R est un moyen de marquage permettant la révélation de la substance enzymatique à identifier ou doser; et

(ii) leurs sels d'addition.

2. Composé suivant la revendication 1, caractérisé en ce que Q est un reste de polyéthylèneglycol de formule $-O[CH_2CH_2O]_n-$ ayant un poids moléculaire moyen de 60-1000.

3. Composé suivant la revendication 1, caractérisé en ce que Q est un reste de polyéthylèneglycol de formule $-O[CH_2CH_2O]_n-$ ayant un poids moléculaire moyen de 200-800.

4. Composé suivant la revendication 1, caractérisé en ce que B est un reste de monoaminoacide choisi parmi l'ensemble constitué par les restes :
   - naturels non basiques dits "neutres" tels que notamment Ala, β-Ala, Cys, Gln, 4Hyp, 3Hyp, Leu, Met, Nle, Nva, Phe, Pro, MeGly, Ser, Tyr, Val, où le groupe OH latéral des restes 4Hyp, 3Hyp, Ser, Thr, Tyr est protégé ou non par un groupe protecteur éther ou ester, et où le groupe SH latéral du reste Cys est protégé ou non par un groupe protecteur thioéther ou thioester;
   - naturels acides tels que notamment Asp, β-Asp, Glu, γ-Glu, dans lesquels la fonction acide latérale est libre ou bloquée sous forme amide ou ester, notamment sous forme d'ester benzylique ou t-butylique;
   - naturels initialement basiques dans lesquels la ou les fonctions basiques latérales n'entrant pas dans la liaison peptidique sont bloquées par un groupe acide convenable éliminant pratiquement le caractère basique tel que Boc, Cbo, Adoc, Aoc, Fmoc, Foc, Iboc, Z, Z(p-Cl), Z(p-OMe), lesdits restes d'aminoacide étant notamment les restes Arg, Lys, His et Orn convenablement bloqués notamment par un dit groupe acide convenable;
   - synthétiques non basiques dits "neutres" tels que notamment ACC, AHX, Aib, ATC, Aze, Abu, 4Abu, CHA, CHG, CHT (où le groupe OH latéral est protégé le cas échéant sous forme éther ou ester), Cle, Phg, Pip, Pyr;
   - synthétiques initialement basiques dans lesquels chaque fonction basique latérale est protégée par un groupe convenable éliminant substantiellement le caractère basique, notamment le reste Dbu, où la fonction basique latérale est bloquée par un groupe tel que Boc, Cbo, Adoc, Aoc, Fmoc, Foc, Iboc, Z, Z(p-Cl) et Z(p-OMe).

5. Composé suivant la revendication 1, caractérisé en ce que B est ACC, Aib, Ala, ATC, Asp, Aze, But, CHA, CHG, Cle, Glu, Gly, 4Hyp, 3Hyp, Ile, Leu, Met, Nle, Nva, Phe, Pip, Pro, MeGly, Ser, Thr, Tyr, Val, β-Ala, un reste CHT, 4Hyp, 3Hyp, Ser, Thr, Tyr dans lequel le groupe OH latéral est protégé sous forme éther ou ester, ou un reste Asp ou Glu dans lequel le groupe OH de la fonction acide carboxylique latérale est remplacé par un groupe $OR_3$, $NH_2$, $NHR_4$ ou $NR_4R_5$ où $R_3$ est alkyle en $C_1$-$C_4$ (de préférence Me, Et, iBu, Bu), hydroxyalkyle en $C_1$-$C_4$ (de préférence $CH_2CH_2OH$), cycloalkyle en $C_3$-$C_6$ (tel que cyclopropyle, cyclopentyle ou cyclohexyle) ou ω-aminoalkyle [où le fragment alkyle est en $C_2$-$C_4$ et où le groupe amino peut être monoalkylé ($NHR_4$), dialkylé ($NR_4R_5$) ou inclus dans un cycle ($NR_4R_5$ = groupe hétérocyclique)], $R_4$ et $R_5$, identiques ou différents, représentent chacun un groupe alkyle en $C_1$-$C_4$, $R_4$ et $R_5$ considérés ensemble pouvant former avec l'atome d'azote, auquel ils sont liés, un groupe N-hétérocyclique choisi parmi l'ensemble constitué par les groupes pyrrolidino, pipéridino, morpholino, thiomorpholino, pipérazino, 4-(2-hydroxyéthyl)pipérazino, 4-méthylpipérazino, 4-(4-chlorophényl)pipérazino et hexaméthylèneimino.

6. Composé suivant la revendication 1, caractérisé en ce que B est choisi parmi l'ensemble constitué par

(i) les restes Aib, L-Ala, β-Ala, L-ATC, L-Aze, L-Abu, L-CHA, L-CHG, L-CHT, Cle, Gly, L-4Hyp, L-3Hyp, L-Asp, L-Glu, L-Ile, L-Leu, L-Nle, L-Nva, L-Phe, L-Pip, L-Pro, MeGly, L-Ser, L-Thr, L-Tyr et L-Val,

(ii) les restes L-CHT, L-4Hyp, L-3Hyp, L-Ser, L-Thr et L-Tyr où le groupe OH latéral est protégé sous la forme d'un groupe ester ou éther,
(iii) les restes L-Arg, L-Lys, L-His, L-Orn et L-Dbu où le groupe basique latéral est substitué par un groupe approprié pour éliminer substantiellement le caractère basique dudit groupe latéral, ledit groupe approprié provenant d'un groupe acide, notamment Boc, Cbo, Adoc, Aoc, Fmoc, Foc, Iboc, Z, Z(p-Cl) et Z(p-OMe)
(iv) les restes L-Asp ou L-Glu où le groupe acide carboxylique latéral est estérifié ou amidifié.

**7.** Composé suivant la revendication 1, caractérisé en ce que B est un reste peptidique, l'aminoacide comportant l'extrémité N-terminale dudit reste peptidique, qui est liée au groupe Q par l'intermédiaire d'un pont carbonyle, est un reste $\alpha$-aminoacide choisi parmi l'ensemble constitué par
(i) les restes $\alpha$-aminoacides dépourvus d'atomes de carbone asymétrique, tels que Aib, Cle et Gly, et
(ii) les restes $\alpha$-aminoacides présentant un atome de carbone asymétrique, et étant de configuration D ou L,
le ou les autres restes aminoacides de la chaîne peptidique B étant de préférence (a) Aib, Cle, Gly ou (b) $\alpha$-aminoacide de configuration L.

**8.** Composé suivant l'une quelconque des revendications 1 et 7, caractérisé en ce que B est un reste dipeptidique ou tripeptidique.

**9.** Composé suivant la revendication 1, caractérisé en ce qu'il est un bipeptide symétrique choisi parmi l'ensemble constitué par
(i) les composés de formule

$$pNA\text{-}Arg\text{-}B\text{-}CO\text{-}O[CH_2CH_2O]_m\text{-}CO\text{-}B\text{-}Arg\text{-}pNA \qquad (Ia)$$

dans laquelle
B est une simple liaison; un reste Aib, Cle, Gly ou un reste de monoaminoacide de configuration L ; ou un reste di- ou tripeptidique dans lequel le reste aminoacide comportant l'extrémité N-terminale de la chaîne peptidique liée au pont carbonyle CO, est Aib, Cle, Gly ou $\alpha$-aminoacide de configuration L ou D, le ou les autres restes aminoacides de ladite chaîne peptidique étant un ou des restes Aib, Cle, Gly ou $\alpha$-aminoacide de configuration L;
n est un nombre entier supérieur ou égal à 1 et tel que le poids moléculaire moyen du groupe $O[CH_2CH_2O]_n$ soit compris entre 60 et 600 environ; et,
(ii) leurs sels d'addition.

**10.** Composé suivant la revendication 1, caractérisé en ce qu'il est choisi parmi l'ensemble constitué par
(a) $PEG_{200}$ $(CO\text{-}Gly\text{-}L\text{-}Pro\text{-}L\text{-}Arg\text{-}pNA)_2$
(b) $PEG_{600}$ $(CO\text{-}Gly\text{-}L\text{-}Pro\text{-}L\text{-}Arg\text{-}pNA)_2$
(c) $PEG_{200}$ $(CO\text{-}D\text{-}Glu(OBzl)\text{-}L\text{-}Pro\text{-}L\text{-}Arg\text{-}pNA)_2$
(d) $PEG_{200}$ $(CO\text{-}D\text{-}Nle\text{-}L\text{-}CHA\text{-}L\text{-}Arg\text{-}pNA)_2$
(e) $PEG_{200}$ $(CO\text{-}D\text{-}Leu\text{-}Gly\text{-}L\text{-}Arg\text{-}pNA)_2$
(f) $PEG_{200}$ $(CO\text{-}D\text{-}CHA\text{-}Gly\text{-}L\text{-}Arg\text{-}pNA)_2$
(g) $PEG_{300}$ $(CO\text{-}D\text{-}Leu\text{-}Gly\text{-}L\text{-}Arg\text{-}pNA)_2$
(h) $PEG_{400}$ $(CO\text{-}D\text{-}Leu\text{-}Gly\text{-}L\text{-}Arg\text{-}pNA)_2$
(i) $PEG_{200}$ $(CO\text{-}D\text{-}Nle\text{-}Gly\text{-}L\text{-}Arg\text{-}pNA)_2$, et
(j) leurs sels d'addition.

**11.** Procédé de détermination d'un enzyme intervenant au niveau de l'hémostase, caractérisé en ce que l'on met en contact dans un milieu biologique aqueux une quantité donnée d'un composé de formule I ou de l'un de ses sels d'addition selon la revendication 1 avec un échantillon à tester contenant ledit enzyme.

**12.** Procédé selon la revendication 11, caractérisé en ce que l'on met en contact dans un milieu biologique aqueux une quantité donnée d'un composé de formule I ou l'un de ses sels d'addition selon la revendication 1 avec un échantillon à tester contenant ledit enzyme, pendant au moins 0,25 h, à une température comprise entre 15 et 40 °C.

**13.** Procédé de préparation d'un composé de formule I et de ses sels d'addition selon la revendication 1, ledit procédé étant caractérisé en ce qu'il comprend la réaction d'un composé aminoacide ou peptidique de formule :

H-B-A-R (IV)

où B, A et R sont définis comme indiqué dans la revendication 1 ci-dessus, avec un dihalogénoformiate de polyalkylèneglycol de formule :

Hal-CO-Q-CO-Hal (V)

dans laquelle Hal représente un atome d'halogène, notamment F, Cl, Br et, de préférence, Cl, et Q est défini comme indiqué dans la revendication 1 ci-dessus.

**14.** Procédé suivant la revendication 13, caractérisé en ce que l'on fait réagir 2 moles de IV avec 1 à 1,3 moles de V dans un solvant inerte, à une température de 0 à 40 °C, en présence d'une amine tertiaire accepteur de protons, selon un rapport molaire amine tertiaire/composé IV de 1,7/1 à 3,6/1.

**15.** Nécessaire de dosage pour la détermination d'enzymes intervenant au niveau de l'hémostase, caractérisé en ce qu'il comprend au moins un composé choisi parmi l'ensemble constitué par les composés de formule I et leurs sels d'addition selon la revendication 1.

## Claims

**1.** A compound belonging to the family of amino acids and peptides containing an amino acid residue bonded to a polyalkylene glycol residue, said compound being selected from the group consisting of
(i) the symmetrical amino acids and peptides of the formula

R-A-B-CO-Q-CO-B-A-R (I)

in which
Q is a polyalkylene glycol residue selected from the polyethylene glycol and polypropylene glycol residues of the formula

$-O[CH(X)CH_2O]_n-$

in which X is H or Me and n is an integer greater than or equal to 1,
B is a single bond, a monoamino acid residue or a peptide chain comprising from 2 to 9 monoamino acid residues,
A is a basic monoamino acid residue selected from the group consisting of $\alpha$-amino acid residues containing a basic group on a side-chain, and
R is a labeling means making it possible to develop the enzyme substance to be identified or assayed; and
(ii) their addition salts.

**2.** A compound according to claim 1 wherein Q is a polyethylene glycol residue of the formula $-O-[CH_2CH_2O]_n-$ having an average molecular weight of 60-1000.

**3.** A compound according to claim 1 wherein Q is a polyethylene glycol residue of the formula $-O-[CH_2CH_2O]_n-$ having an average molecular weight of 200-800.

**4.** A compound according to claim 1 wherein B is a monoamino acid residue selected from the group consisting of the residues derived from the following amino acids:
- so-called "neutral" non-basic natural amino acids, such as, in particular, Ala, $\beta$-Ala, Cys, Gln, 4Hyp, 3Hyp, Leu, Met, Nle, Nva, Phe, Pro, MeGly, Ser, Tyr and Val, where the OH side-group of the 4Hyp, 3Hyp, Ser, Thr and Tyr residues may or may not be protected by an ether or ester protecting group, and where the SH side-group of the Cys residue may or may not be protected by a thioether or thioester protecting group;

- acidic natural amino acids, such as, in particular, Asp, $\beta$-Asp, Glu and $\gamma$-Glu, in which the acid side-group is free or blocked in the form of an amide or ester, especially in the form of the benzyl or t-butyl ester;
- initially basic natural amino acids in which the basic side-group or side-groups not forming part of the peptide linkage are blocked by an appropriate acid group which practically eliminates the basic character, such as Boc, Cbo, Adoc, Aoc, Fmoc, Foc, Iboc, Z, Z(p-Cl) and Z(p-OMe), said amino acid residues being especially Arg, Lys, His and Orn residues appropriately blocked by one of said appropriate acid groups in particular;
- so-called "neutral" non-basic synthetic amino acids, such as, in particular, ACC, AHX, Aib, ATC, Aze, Abu, 4Abu, CHA, CHG, CHT (where the OH side-group is protected, if appropriate, in the form of an ether or ester), Cle, Phg, Pip and Pyr; and
- initially basic synthetic amino acids in which each basic side-group is protected by an appropriate group which substantially eliminates the basic character, especially the Dbu residue, where the basic side-group is blocked by a group such as Boc, Cbo, Adoc, Aoc, Fmoc, Foc, Iboc, Z, Z(p-Cl) and Z(p-OMe).

**5.** A compound according to claim 1 wherein B is ACC, Aib, Ala, ATC, Asp, Aze, But, CHA, CHG, Cle, Glu, Gly, 4Hyp, 3Hyp, Ile, Leu, Met, Nle, Nva, Phe, Pip, Pro, MeGly, Ser, Thr, Tyr, Val, $\beta$-Ala, a CHT, 4Hyp, 3Hyp, Ser, Thr or Tyr residue in which the OH side-group is protected in the form of an ether or ester, or an Asp or Glu residue in which the OH group of the carboxylic acid side-group has been replaced with a group $OR_3$, $NH_2$, $NHR_4$ or $NR_4R_5$, in which $R_3$ is $C_1$-$C_4$ alkyl (preferably Me, Et, iBu, Bu), $C_1$-$C_4$ hydroxyalkyl (preferably $CH_2CH_2OH$), $C_3$-$C_6$ cycloalkyl (such as cyclopropyl, cyclopentyl or cyclohexyl) or $\omega$-aminoalkyl [in which the alkyl fragment is $C_2$-$C_4$ and in which the amino group can be monoalkylated ($NHR_4$), dialkylated ($NR_4R_5$) or included in a ring ($NR_4R_5$ = heterocyclic group)] and $R_4$ and $R_5$, which are identical or different, are each a $C_1$-$C_4$ alkyl group, it being possible for $R_4$ and $R_5$, taken together, to form, with the nitrogen atom to which they are bonded, an N-heterocyclic group selected from the group consisting of pyrrolidino, piperidino, morpholino, thiomorpholino, piperazino, 4-(2-hydroxyethyl)piperazino, 4-methylpiperazino, 4-(4-chlorophenyl)piperazino and hexamethyleneimino groups.

**6.** A compound according to claim 1 wherein B is selected from the group consisting of
(i) Aib, L-Ala, $\beta$-Ala, L-ATC, L-Aze, L-Abu, L-CHA, L-CHG, L-CHT, Cle, Gly, L-4Hyp, L-3Hyp, L-Asp, L-Glu, L-Ile, L-Leu, L-Nle, L-Nva, L-Phe, L-Pip, L-Pro, MeGly, L-Ser, L-Thr, L-Tyr and L-Val residues,
(ii) L-CHT, L-4Hyp, L-3Hyp, L-Ser, L-Thr and L-Tyr residues in which the OH side-group is protected in the form of an ester or ether group,
(iii) L-Arg, L-Lys, L-His, L-Orn and L-Dbu residues in which the basic side-group is substituted by an appropriate group for substantially eliminating the basic character of said side-group, said appropriate group being derived from an acid group, especially Boc, Cbo, Adoc, Aoc, Fmoc, Foc, Iboc, Z, Z(p-Cl) and Z(p-OMe), and
(iv) L-Asp or L-Glu residues in which the carboxylic acid side-group is esterified or amidated.

**7.** A compound according to claim 1 wherein B is a peptide residue and the amino acid containing the N-terminal end of said peptide residue which is bonded to the group Q via a carbonyl bridge is an $\alpha$-amino acid residue selected from the group consisting of
(i) $\alpha$-amino acid residues devoid of asymmetric carbon atoms, such as Aib, Cle and Gly, and
(ii) $\alpha$-amino acid residues containing an asymmetric carbon atom and having the D or L configuration, the other amino acid residue or residues of the peptide chain B preferably being (a) Aib, Cle or Gly, or (b) an $\alpha$-amino acid having the L configuration.

**8.** A compound according to claim 1 or claim 7 wherein B is a dipeptide or tripeptide residue.

**9.** A compound according to claim 1 which is a symmetrical bipeptide selected from the group consisting of
(i) the compounds of the formula

$$\text{pNA-Arg-B-CO-O}[CH_2CH_2O]_m\text{-CO-B-Arg-pNA} \qquad (Ia)$$

in which

B is a single bond; an Aib, Cle or Gly residue or a monoamino acid residue having the L configuration; or a di- or tri-peptide residue in which the amino acid residue containing the N-terminal end of the peptide chain which is bonded to the carbonyl bridge, CO, is Aib, Cle, Gly or an $\alpha$-amino acid having the L or D configuration, the other amino acid residue or residues of said peptide chain being one or more Aib, Cle or Gly residues or $\alpha$-amino acid residues having the L configuration; and

n is an integer greater than or equal to 1, such that the average molecular weight of the group $O[CH_2CH_2O]_n$ is between about 60 and 600; and

(ii) their addition salts.

**10.** A compound according to claim 1 which is selected from the group consisting of

(a) $PEG_{200}$(CO-Gly-L-Pro-L-Arg-pNA)$_2$,
(b) $PEG_{600}$(CO-Gly-L-Pro-L-Arg-pNA)$_2$,
(c) $PEG_{200}$(CO-D-Glu(OBzl)-L-Pro-L-Arg-pNA)$_2$,
(d) $PEG_{200}$(CO-D-Nle-L-CHA-L-Arg-pNA)$_2$,
(e) $PEG_{200}$(CO-D-Leu-Gly-L-Arg-pNA)$_2$,
(f) $PEG_{200}$(CO-D-CHA-Gly-L-Arg-pNA)$_2$,
(g) $PEG_{300}$(CO-D-Leu-Gly-L-Arg-pNA)$_2$,
(h) $PEG_{400}$(CO-D-Leu-Gly-L-Arg-pNA)$_2$,
(i) $PEG_{200}$(CO-D-Nle-Gly-L-Arg-pNA)$_2$ and
(j) their addition salts.

**11.** A method of determining an enzyme involved in hemostasis, wherein a given amount of a compound of formula I or of one of its addition salts according to claim 1 is brought into contact, in an aqueous biological medium, with a test sample containing said enzyme.

**12.** A method according to claim 11 wherein a given amount of a compound of formula I or of one of its addition salts according to claim 1 is brought into contact, in an aqueous biological medium, with a test sample containing said enzyme for at least 0.25 h at a temperature of between 15 and 40 °C.

**13.** A method preparing a compound of formula I and its addition salts according to claim 1, said method comprising reacting an amino acid or peptide compound of the formula

H-B-A-R (IV)

in which B, A and R are defined as indicated in claim 1 above, with a polyalkylene glycol dihalogenoformate of the formula

Hal-CO-Q-CO-Hal (V)

in which Hal is a halogen atom, especially F, Cl or Br and preferably Cl, and Q is defined as indicated in claim 1 above.

**14.** A method according to claim 13 wherein 2 mol of IV are reacted with 1 to 1.3 mol of V in an inert solvent at a temperature of 0 to 40 °C, in the presence of a tertiary amine as a proton acceptor, in a molar ratio tertiary amine/compound IV of 1.7/1 to 3.6/1.

**15.** An assay kit for the determination of enzymes involved in hemostasis, which comprises at least one compound selected from the group consisting of the compounds of formula I and their addition salts according to claim 1.

**Patentansprüche**

**1.** Verbindung, die zu der Familie der Aminosäuren und der Peptide gehört, die einen an einen Polyalkylenglykolrest gebundenen Aminosäurerest aufweist und die dadurch gekennzeichnet ist, daß sie aus der Gruppe ausgewählt ist, die von

(i) symmetrischen Aminosäuren und Peptiden der Formel

R-A-B-CO-Q-CO-B-A-R (I)

in der
Q ein Rest eines Polyalkylenglykols, ausgewählt aus den Resten von Polyethylenglykol und Polypropylenglykol der Formel

$-O[CH(X)CH_2O]_n-$

wobei X H oder Me und n eine ganze Zahl größer als oder gleich 1 ist,
B eine Einfachbindung, ein Rest einer Monoaminosäure oder eine 2 bis 9 Monoaminosäuren enthaltende Peptidkette,
A ein Rest einer basischen Monoaminosäure, ausgewählt aus der die Reste von eine basische Gruppe an einer Seitenkette tragenden alpha-Aminosäuren enthaltenden Gruppe, und
R ein den Nachweis der zu identifizierenden oder zu dosierenden enzymatischen Substanz ermöglichende Markierungsmittel
bedeuten, sowie
(ii) Additionssalzen derselben
gebildet ist.

**2.** Verbindung nach Anspruch 1, dadurch gekennzeichnet, daß Q der Rest eines Polyethylenglykols der Formel $-O[CH_2CH_2O]_n-$ mit einem mittleren Molekulargewicht von 60 - 1000 ist.

**3.** Verbindung nach Anspruch 1, dadurch gekennzeichnet, daß Q der Rest eines Polyethylenglykols der Formel $-O[CH_2CH_2O]_n-$ mit einem mittleren Molekulargewicht von 200 - 800 ist.

**4.** Verbindung nach Anspruch 1, dadurch gekennzeichnet, daß B der Rest einer Monoaminosäure ist, der aus einer Gruppe ausgewählt ist, die von den Resten

- natürliche, nichtbasische, "neutral" genannte, wie insbesondere Ala, beta-Ala, Cys, Gln, 4Hyp, 3Hyp, Leu, Met, Nle, Nva, Phe, Pro, MeGly, Ser, Tyr, Val, wobei die seitenständige Gruppe OH der Reste 4Hyp, 3Hyp, Ser, Thr, Tyr durch eine Ether- oder Ester-Schutzgruppe geschützt oder ungeschützt ist, und wobei die Seitengruppe SH des Restes Cys durch eine Thioether- oder Thioester-Schutzgruppe geschützt oder ungeschützt ist;
- natürliche Säuren wie insbesondere Asp, beta-Asp, Glu, gamma-Glu, in denen die seitenständige Säurefunktion frei oder in Form eines Amids oder Esters blockiert ist, insbesondere in Form eines Benzyl- oder t-Butylesters;
- natürliche, ursprünglich basische, in denen die seitenständige(n) basische(n) Funktion(en) die nicht eine Peptidbindung eingeht bzw. eingehen, durch eine geeignete Säuregruppe blockiert sind, welche praktisch den basischen Charakter eliminiert, wie Boc, Cbo, Adoc, Aoc, Fmoc, Foc, Iboc, Z, Z(p-Cl), Z(p-OMe), wobei die genannten Reste der Aminosäure insbesondere die Reste Arg, Lys, His und Orn, geeignet blockiert insbesondere durch eine geeignete, genannte Säuregruppe, sind;
- synthetische, nicht-basische, "neutral" genannte wie insbesondere ACC, AHX, Aib, ATC, Aze, Abu, 4Abu, CHA, CHG, CHT (wobei die seitenständige OH-Gruppe gegebenenfalls in Form eines Ethers oder Esters geschützt ist), Cle, Phg, Pip, Pyr;
- synthetische, ursprünglich basische, in denen jede seitenständige basische Funktion durch eine geeignete Gruppe geschützt ist, die im wesentlichen den basischen Charakter eliminiert, insbesondere der Rest Dbu, wobei die seitenständige basische Funktion durch eine Gruppe wie Boc, Cbo, Adoc, Aoc, Fmoc, Foc, Iboc, Z, Z(p-Cl) und Z(p-OMe) blockiert ist,

gebildet ist.

**5.** Verbindung nach Anspruch 1, dadurch gekennzeichnet, daß B ACC, Aib, Ala, ATC, Asp, Aze, But, CHA, CHG, Cle, Glu, Gly, 4Hyp, 3Hyp, Ile, Leu, Met, Nle, Nva, Phe, Pip, Pro, MeGly, Ser, Thr, Tyr, Val, beta-Ala, ein Rest CHT, 4Hyp, 3Hyp, Ser, Thr, Tyr ist, wobei die seitenständige Gruppe OH in Form eines Ethers oder Esters geschützt ist, oder ein Rest Asp oder Glu ist, in dem die Gruppe OH der seitenständigen Carbonsäurefunktion durch eine Gruppe $OR_3$, $NH_2$, $NHR_4$ oder $NR_4R_5$ substituiert ist, wobei $R_3$ $C_1$-$C_4$-Alkyl (vorzugsweise Me, Et, iBu, Bu), $C_1$-$C_4$-Hydroxyalkyl (vorzugsweise $CH_2CH_2OH$), $C_3$-$C_6$-Cycloalkyl (wie Cyclopropyl, Cyclopentyl oder Cyclohexyl) oder omega-Aminoalkyl [wobei der Alkylrest $C_2$-$C_4$ ist oder wobei die Aminogruppe monoalkyliert ($NHR_4$), dialkyliert ($NR_4R_5$) oder in einen

Ring ($NR_4R_5$ = heterozyklische Gruppe) eingeschlossen ist], $R_4$ und $R_5$, die gleich oder verschieden sein können, jeweils eine $C_1$-$C_4$-Alkylgruppe bedeuten und $R_4$ und $R_5$ zusammen mit dem Stickstoffatom, an das sie gebunden sind, eine N-heterozyklische Gruppe bilden können, die aus der Gruppe ausgewählt ist, welche von den Pyrrolidino-, Piperidino-, Morpholino-, Thiomorpholino-, Piperazino-, 4-(2-Hydroxyethyl)piperazino-, 4-Methylpiperazino-, 4-(4-Chlorophenyl)piperazino- und Hexamethyleniminо-Gruppen gebildet wird.

**6.** Verbindung nach Anspruch 1, dadurch gekennzeichnet, daß B aus der Gruppe ausgewählt ist, die von
(i) den Resten Aib, L-Ala, beta-Ala, L-ATC, L-Aze, L-Abu, L-CHA, L-CHG, L-CHT, Cle, Gly, L-4Hyp, L-3Hyp, L-Asp, L-Glu, L-Ile, L-Leu, L-Nle, L-Nva, L-Phe, L-Pip, L-Pro, MeGly, L-Ser, L-Thr, L-Tyr und L-Val,
(ii) den Resten L-CHT, L-4Hyp, L-3Hyp, L-Ser, L-Thr und L-Tyr, wobei die seitenständige OH-Gruppe in Form einer Ester- oder Ethergruppe geschützt ist,
(iii) den Resten L-Arg, L-Lys, L-His, L-Orn und L-Dbu, wobei die seitenständige basische Gruppe durch eine geeignete Gruppe substituiert ist, um im wesentlichen den basischen Charakter dieser seitenständigen Gruppe zu eliminieren und wobei die genannte geeignete Gruppe von einer Säuregruppe abgeleitet ist, insbesondere Boc, Cbo, Adoc, Aoc, Fmoc, Foc, Iboc, Z, Z(p-Cl) und Z(p-OMe), und
(iv) den Resten L-Asp oder L-Glu, wobei die seitenständige Carbonsäuregruppe verestert oder amidiert ist,
gebildet ist.

**7.** Verbindung nach Anspruch 1, dadurch gekennzeichnet, daß B ein Peptidrest ist, wobei die das N-terminale Ende dieses Peptidrestes tragende Aminosäure, die mit der Gruppe Q mit Hilfe einer Carbonylbrücke verbunden ist, ein alpha-Aminosäurerest ist, der aus einer Gruppe ausgewählt ist, die von
(i) den Resten von alpha-Aminosäuren ohne asymmetrische Kohlenstoffatome, wie Aib, Cle und Gly, und
(ii) den Resten von alpha-Aminosäuren mit einem asymmetrischen Kohlenstoffatom, die sich in der D- oder L-Konfiguration befinden,
gebildet ist, wobei der bzw. die andere(n) Rest(e) der Aminosäuren der Peptidkette B vorzugsweise (a) Aib, Cle, Gly oder (b) alpha-Aminosäuren der Konfiguration L sind.

**8.** Verbindung nach einem jeden der Ansprüche 1 und 7, dadurch gekennzeichnet, daß B ein Dipeptid- oder Tripeptid-Rest ist.

**9.** Verbindung nach Anspruch 1, dadurch gekennzeichnet, daß sie ein symmetrisches Bipeptid ist, das aus der Gruppe ausgewählt ist, die von
(i) den Verbindungen der Formel

$$pNA\text{-}Arg\text{-}B\text{-}CO\text{-}O[CH_2CH_2O]_m\text{-}CO\text{-}B\text{-}Arg\text{-}pNA \qquad (Ia)$$

in der
B eine Einfachbindung, ein Rest Aib, Cle, Gly oder ein Rest einer Monoaminosäure der Konfiguration L oder ein Di- oder Tripeptid-Rest ist, in dem der Aminosäurerest,
der das N-terminale Ende der Peptidkette mit einem Brückencarbonyl CO verknüpft trägt, Aib, Cle, Gly oder eine alpha-Aminosäure der Konfiguration L oder D ist, wobei der bzw. die andere(n) Rest(e) der Aminosäuren der genannten Peptidkette ein Rest oder Reste Aib, Cle, Gly oder Aminosäure der Konfiguration L ist bzw. sind; und
n eine ganze Zahl größer als oder gleich 1 und so groß ist, daß das mittlere Molekulargewicht der Gruppe $O[CH_2CH_2O]_n$ etwa zwischen 60 und 600 liegt, und

(ii) ihren Additionssalzen
gebildet ist.

**10.** Verbindung nach Anspruch 1, dadurch gekennzeichnet, daß sie aus der Gruppe ausgewählt ist, die von
(a) $PEG_{200}$ (CO-Gly-L-Pro-L-Arg-pNA)$_2$
(b) $PEG_{600}$ (CO-Gly-L-Pro-L-Arg-pNA)$_2$
(c) $PEG_{200}$ (CO-D-Glu(OBzl)-L-Pro-L-Arg-pNA)$_2$

(d) $PEG_{200}$ (CO-D-Nle-L-CHA-L-Arg-pNA)$_2$
(e) $PEG_{200}$ (CO-D-Leu-Gly-L-Arg-pNA)$_2$
(f) $PEG_{200}$ (CO-D-CHA-Gly-L-Arg-pNA)$_2$
(g) $PEG_{300}$ (CO-D-Leu-Gly-L-Arg-pNA)$_2$
(h) $PEG_{400}$ (CO-D-Leu-Gly-L-Arg-pNA)$_2$
(i) $PEG_{200}$ (CO-D-Nle-Gly-L-Arg-pNA)$_2$, und
(j) ihren Additionssalzen
gebildet ist.

**11.** Verfahren zur Bestimmung eines auf dem Niveau der Hämostase wirkenden Enzyms, dadurch gekennzeichnet, daß man in einem wäßrigen biologischen Milieu eine definierte Menge einer Verbindung der Formel I oder eines Additionssalzes derselben nach Anspruch 1 mit einer zu untersuchenden, das genannte Enzym enthaltenden Probe zusammenbringt.

**12.** Verfahren nach Anspruch 11, dadurch gekennzeichnet, daß man in einem wäßrigen, biologischen Milieu eine definierte Menge einer Verbindung der Formel I oder eines Additionssalzes derselben gemäß Anspruch 1 mit einer zu untersuchenden, das genannte Enzym enthaltenden Probe wenigstens 0,25 Stunden bei einer Temperatur zwischen 15 und 40 °C zusammenbringt.

**13.** Verfahren zur Herstellung einer Verbindung der Formel I und Additionssalzen derselben nach Anspruch 1, dadurch gekennzeichnet, daß es die Reaktion einer Aminosäure- oder Peptidverbindung der Formel

H-B-A-R (IV)

in der B, A und R wie in dem obigen Anspruch 1 angegeben definiert sind, mit einem Dihalogenformiat eines Polyalkylenglykols der Formel

Hal-CO-Q-CO-Hal (V)

in der Hal ein Halogenatom, insbesondere F, Cl und Br sowie vorzugsweise Cl bedeutet und Q wie in dem obigen Anspruch 1 angegeben definiert ist, umfaßt.

**14.** Verfahren nach Anspruch 13, dadurch gekennzeichnet, daß man zwei Mol IV mit 1 bis 1,3 Mol V in einem inerten Lösemittel reagieren läßt, und zwar bei einer Temperatur von 0 bis 40 °C in Gegenwart eines tertiären Amins als Protonenakzeptor, gemäß einem molaren Verhältnis von tertiärem Amin/Verbindung IV von 1,7/1 bis 3,6/1.

**15.** Dosierungskit für die Bestimmung von Enzymen, die auf dem Niveau der Hämostase wirken, dadurch gekennzeichnet, daß es mindestens eine Verbindung enthält, die aus der Gruppe ausgewählt ist, welche von den Verbindungen der Formel I und ihren Additionssalzen gemäß Anspruch 1 gebildet ist.